# EUROPEAN PATENT SPECIFICATION

(11) **EP 2 709 722 B1**
(45) Date of publication and mention of the grant of the patent: **10.11.2021**
(21) Application number: 12724285.7
(22) Date of filing: 15.05.2012
(51) Int. Cl.: A43B 3/00, A61N 1/36, A61N 1/04, A43B 1/00, A43B 7/14, A61B 5/00

(54) **FOOTWEAR PRODUCT FOR FUNCTIONAL ELECTRICAL STIMULATION**
SCHUHWERK ZUR FUNKTIONELLEN ELEKTROSTIMULATION
PRODUIT CHAUSSANT POUR STIMULATION ÉLECTRIQUE FONCTIONNELLE

(30) Priority: 17.05.2011 DK 201170244
(43) Date of publication of application: 26.03.2014
(73) Proprietor: Nordic - NeuroSTIM ApS, 9220 Aalborg Ö (DK)
(72) Inventor: EMBORG, Jonas, 3660 Stenløse (DK); ANDERSEN, Ole Kæseler, 9520 Skørping (DK); SPAICH, Erika Geraldina, 9270 Klarup (DK)
(74) Representative: AWA Sweden AB
(86) International application number: PCT/DK2012/050171
(87) International publication number: WO 2012/155917

(56) References cited:
- US-A1- 2004 173 220
- Jonas Emborg: "Modulation of the nociceptive withdrawal reflex and its use in rehabilitation of gait of stroke patient", , 1 January 2010 (2010-01-01), pages 1-47, XP055045108, ISBN: 978-8-77-094075-7 Retrieved from the Internet: URL:http://vbn.aau.dk/files/44356316/PhDTh esis_JonasEmborg.pdf [retrieved on 2012-11-21]
- JONAS EMBORG ET AL: "Withdrawal reflexes examined during human gait by ground reaction forces: site and gait phase dependency", MEDICAL & BIOLOGICAL ENGINEERING & COMPUTING, SPRINGER, BERLIN, DE, vol. 47, no. 1, 1 October 2008 (2008-10-01), pages 29-39, XP019835300, ISSN: 1741-0444
- OLE K ANDERSEN ET AL: "Reflex receptive fields for human withdrawal reflexes elicited by non-painful and painful electrical stimulation of the foot sole", CLINICAL NEUROPHYSIOLOGY, vol. 112, no. 4, 1 April 2001 (2001-04-01) , pages 641-649, XP055045123, ISSN: 1388-2457, DOI: 10.1016/S1388-2457(01)00485-0

## Description

### FIELD OF THE INVENTION

The invention relates to the field of medical devices, specifically the invention provides a footwear product for use in Functional Electrical Stimulation (FES) or Functional Electrical Therapy (FET).

### BACKGROUND OF THE INVENTION

Functional Electric Stimulation FES or Functional Electric Therapy FET is well known to assist patients in walking. For example the Ph.D. thesis "Modulation of the nociceptive withdrawal reflex and its use in rehabilitation of gait of stroke patients", Aalborg University 2009, by one of the inventors, Jonas Emborg describes reflex-based gait traning where a painful electric stimulation on the patient's foot sole helps the patient to move his/her leg and thus initiate a step due to the evoked withdrawal reflex. This provides a helpfule tool to enable gait training of patients being partially or completely immobile due to a hemiparetic condition caused by a stroke or traumatic brain injury which has resulted in damage of an area of their brain. Such patients have a significant better chance of rehabilitation if intensive gait training is initiated within 3 months after the stroke. In this early period the brain is especially suited to regenerate/relearn the ability to control the muscles, if the sufficient sensory, learning input is provided, i.e. by completing functionally adequate gait training.

Further references from the inventors are:
- *"Design and test of a novel closed-loop system that exploits the nociceptive withdrawal reflex for swing phase support of the hemiparetic gait",* J. Emborg, Z. Matja 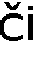 ić, J. D. Bendtsen, E.G. Spaich, I. Cikajlo, N. Goljar, O.K. Andersen. IEEE Transactions on Biomedical Engineering. vol. 58, no. 4, pp. 960-970, Apr. 2011. DOI: 10.1109/TBME.2010.2096507
- *"*Withdrawal reflex responses evoked by repetitive painful stimulation delivered on the sole of the foot during late stance: site, phase, and frequency modulation", E. G. Spaich, J. Emborg, T. Collet, L. Arendt-Nielsen, and O. K. Andersen, Exp. Brain Res., vol. 194, no. 3, pp. 359-368, Apr. 2009
- *"*Withdrawal reflexes examined during human gait by ground reaction forces: site and gait phase dependency", J. Emborg, E. G. Spaich, and O. K. Andersen, Med. Biol. Eng. Comput. 2009; vol. 4, pp. 29-39, Jan. 2009
- "Novel method exploiting the nociceptive withdrawal reflexes in rehabilitation of hemiplegic gait" Emborg, J. ; Bendtsen, J. D. ; Spaich, E. G. ; Andersen, O. K., 2009. s. 84-87 World Congress on Medical Physics and Biomedical Engineering, Munich, Germany, 7-12 September 2009, International Federation for Medical and Biological Engineering Proceedings. 25. IX

Laboratory experiments with the above-mentioned reflex-based gait training can be performed using electric stimulation applied by means of adhesive electrodes manually attached to the patient's foot, e.g. electrodes as used for ECG monitoring can be used. However, for practical application of the method several electrodes are used, and thus in a physiotherapy clinic or at a hospital, time is wasted on mounting and taking off the equipment instead of providing actual gait training for a patient.

### SUMMARY OF THE INVENTION

Therefore, following the above description, there is a need for a product to facilitate practical and easy application of painful electrical stimulations of the person's foot for reflex-based gait training.

The invention is defined by a product according to claim 1 and a system according to claim 14. Preferred embodiments are defined in the dependent claims.

In a first aspect, the invention provides a product shaped and configured to fit onto a person's foot sole, the product comprising
- a platform of a flexible and electrically isolating material,
- a plurality of separate stimulation electrodes each having an adhesive stimulation area of at least 1 cm², wherein the stimulation electrodes are spatially distributed on the platform so as to be able to stimulate different zones of the person's foot sole,
- at least one electrically conducting and adhesive ground plane electrode configured to contact the person's foot, wherein a total area of the ground plane electrode is at least 10 times greater than the area of one stimulation electrode,
- a common electrical interface, electrically connected to the plurality of separate stimulation electrodes and the ground plane electrode, the common electrical interface comprising a plug or socket with externally accessible electrical connectors configured to allow external individual electrical access to the plurality of stimulation electrode areas and to the ground plane electrode, and
- a set of electrical conductors configured to interconnect the plurality of stimulation electrodes and the ground plane electrode with the electrical connectors of the common electrical interface, wherein the set of electrical conductors are configured to withstand a voltage of more than 200 V between the ground plane electrode and the plurality of stimulation electrodes.

Such product, e.g. in the form of a disposable sole for a shoe, is suitable in practical gait training therapy because the stimulation equipment necessary only needs to be plugged onto the common electrical interface of such product to initiate a gait therapy session. Time consuming individual matching and mounting of stimulation electrodes, often several electrodes, is eliminated, since the product can be manufactured in different sizes thus fitting onto the person's foot without any individual electrode positioning considerations. The product needs to be mounted on the foot sole of the person, and the common electrical interface needs to be connected to the control system serving to apply the stimulation signal, e.g. in the form of a portable control device carried in the person's belt or the like which may then be wired or wirelessly connected to a stationary or laptop computer system. It is possible to manufacture the product at a low price, thus allowing the product to be used only once, thereby ensuring a high hygienic level without the need for cleaning of the equipment between two persons using the gait training equipment.

The product may be specific for being used primarily, but not exclusively, in connection with the Functional Electric Therapy, FET. The technology has also potential for being used in relation to a diagnostic tool that quantifies central sensitization in relation to chronic pain. This has the application to diagnose chronic pain patients - a large and until now unsolved problem - by assessing expanded reflex receptive fields via distributed electrical stimulations. Stimulations are delivered to different sites on the sole of the foot with the intent to asses spatial variation in withdrawal reflex sensitivity. Also, here a practical method of mounting several electrodes are needed to reduce the time for donning and doffing equipment.

Furthermore, in embodiments with many spatially separated stimulation electrodes, the best possible electrical stimulation of the individual person becomes possible irrespective of individual spatial differences in persons' sensitivities. The computer algorithm can take into account utilization of the best possible stimulation positions based on a feedback loop indicating the resulting swing of the leg and activating the most appropriate stimulation electrodes accordingly. The fact that the product is capable of handling 200 V, preferably also more than 400 V, or more, allows for efficient powerful stimulation, and the defined area difference between ground plane electrode and stimulation electrodes ensures that the intentional withdrawal reflex provoking sensation is obtained when a suitable electric stimulation signal is applied.

In versions with a special electrical interface plug, a high safety can be obtained since the risk is minimized that wrong equipment is connected to the product.

The product may be in the form of a more or less simple footwear product, and especially the product may be: 1) a replaceable insole arranged to fit into a shoe, 2) a slipper, or 3) a shoe. The platform may form part of a footwear, such as a shoe, or a slipper.

In one embodiment, the product is a replaceable insole arranged to fit into a person's shoe, and wherein the platform comprises an upper layer and a lower layer with the set of electrical conductors arranged between the upper and lower layers, and wherein the stimulation electrodes extend through holes in the upper layer. As explained, such product can be manufactured at a low cost thus allowing one-time use and thereby easy mounting of a new clean product on the foot of each person. Especially, at least the upper layer may be made of a reusable material, such as a sheet of nonwoven material. Such embodiment will allow only a limited waste of material in case of a one-time use product.

The product comprises a plurality of stimulation electrodes being spatially distributed on the platform so as to enable stimulation of different zones of the person's foot sole during normal use. According to an embodiment, the plurality of stimulation electrodes are spatially distributed on the platform so as to enable stimulation of a plurality of different positions of each one of the person's foot sole areas: heel, fore foot, and mid foot. This allows a FET control system to activate the most efficient stimulation positions for obtaining the desired withdrawal reflex and thus e.g. reduce adaptation (habituation) effects.

As already explained, this allows an automated FET system to activate the most appropriate positions according to a feedback loop, thus accounting for individual differences.

In some embodiments, the ground plane extends spatially so as to enable contact to at least one of the person's foot regions: heel of foot sole, fore foot of foot sole, mid foot of foot sole, back of heel, and upper part of foot. It may be preferred that the ground plane electrode extends spatially so as to enable contact to a plurality of said regions of the person's foot. In one specific embodiment, the product comprises a plurality of spatially separated ground plane electrodes, while in other embodiments, the ground plane electrode may extend in several ones of said regions by constituting one large conductive area.

It may be preferred that the platform is substantially foot shaped, such that it fits the shape of a normal foot and thus fits inside existing footwear, in case it is an insole product.

In some embodiments, the product comprises an additional platform element structurally linked to the platform, wherein the additional platform element comprises a stimulation electrode or a ground plane electrode in electrical connection with said common electrical interface. Especially, the additional platform element is connected to the platform so as to enable the stimulation electrode or the ground plane electrode to be in contact with an upper part of or a back of the heel of the person's foot when mounted for normal use. Such additional platform element may be in the form of a flap that can be bent to make contact to the back of the heel or the upper part of the person's foot during normal use.

The product may comprise a sensor arranged to provide a feedback indicative of a person's gait phase upon the person walking when wearing the product, such as a sensor arranged between the platform and an upper layer of the product. Such sensor will allow the product to be used in FET based systems with feedback, where the sensor can be used to provide feedback to the FET algorithm regarding in which gait phase the leg is. This may eliminate the need for further feedback sensors, thus providing easy mounting of the equipment, since no individual fitting is needed, if the sensor is integrated in the product. In specific embodiment, the sensor is electrically connected to one or more electrical connectors within said common electrical interface, such as within the same plug or socket providing the external connection of the stimulation electrodes and the ground plane. This allows very easy connection to a feedback based FET system. Especially, it may be preferred that at least one sensor is spatially arranged in relation to the platform so as to enable sensing of the person's foot touching the ground, when wearing the product. The sensor may comprise an electric switch arranged to switch between an electrically closed and electrically open state upon a person's foot touching the ground, when wearing the product. Such electric switch may be implemented by one of: a force sensitive sensor, an electric switch with an on state and an off state, capacitive transducers, load cells, strain gauges, piezoelectrical force sensors. Simple sensors can be manufactured in low cost and still allow the product to be in the form of a one-time use insole product. The sensor may include one or more advanced sensor technologies, e.g. a laser-based distance sensor to sense distance to the ground, an accelerometer etc., however in such case the manufacturing costs will increase and thus such embodiments are not suited for one-time use.

To further allow a more detailed information about the gait phase, the sensor may comprise a plurality of spatially distributed sensor elements, such as a plurality of electric switches, in different regions of the platform so as to enable sensing of respective areas of the person's foot touching the ground, when wearing the product. Especially, the plurality of sensor elements are electrically connected to respective electrical connectors of the common electrical interface so as to allow external individual electrical access to the plurality of sensor elements via one single plug or socket of the common electrical interface.

The common electrical interface may be implemented by a connector in the form of a plug or socket having a shape different from standard connectors, so as to avoid non-intended connection to external equipment.

The set of electrical conductors may comprise a flexible PCB spatially extending within the platform so as to electrically connect each of the plurality of stimulation electrodes and the respective electrical connectors of the common electrical interface.

An end of the set of electrical conductors may extend away from the platform, so as to allow the plug or socket of the common electrical interface to be placed outside a person's shoe.

In a second aspect, the invention provides a system arranged for gait training, such as for rehabilitation of a person after a stroke, the system comprising
- a product according to the first aspect,
- a stimulation unit arranged for connection to the common electrical interface of the product, wherein the stimulation unit is arranged to generate an electrical stimulation voltage between the ground plane electrode and at least one of the stimulation electrodes, wherein the electric stimulation voltage is high enough to induce a painful sensation on a foot of a person wearing the product with the purpose of provoking the person's withdrawal reflex.

In one embodiment, the system comprises
- a sensor arranged to sense a parameter representative of a movement of the person's leg and to generate a feedback signal accordingly, such as a sensor as described in embodiments of the first aspect, and
- a processor unit operationally connected to the stimulation unit and to the sensor, the processor unit comprising a processor running a control algorithm configured to generate a control signal to the stimulation unit in response to the feedback signal.

According to an example, use of a product according to the first aspect is provided for Functional Electric Stimulation, such as reflex based gait training, such as for rehabilitation of a person after a stroke.

According to another example, use of a product according to the first aspect is provided for diagnostics of central sensitization in relation to chronic pain by assessing expanded reflex receptive fields via distributed electrical stimulation.

It is appreciated that the same advantages and equivalent embodiments apply for the second aspect as mentioned for the first aspect.

### BRIEF DESCRIPTION OF THE DRAWINGS

Embodiments of the invention will be described, by way of example only, with reference to the drawings, in which
Fig. 1 illustrates an example of stimulation electrode and ground plane electrode layout of an insole embodiment,
Figs. 2a and 2b illustrate two views of an insole embodiment with a ground plane electrode and a heel stimulation electrode positioned on flaps on the insole platform,
Figs. 3a and 3b illustrate two views of another insole embodiment with one centrally positioned ground plane electrode,
Fig. 4 illustrates basic parts of a FET system including a product of the present invention,
Fig. 5 illustrates an insole embodiment connected to parts of a FET system, and
Fig. 6 illustrates an example of an implementation of a FET system including an insole according to the invention.

### DESCRIPTION OF EMBODIMENTS

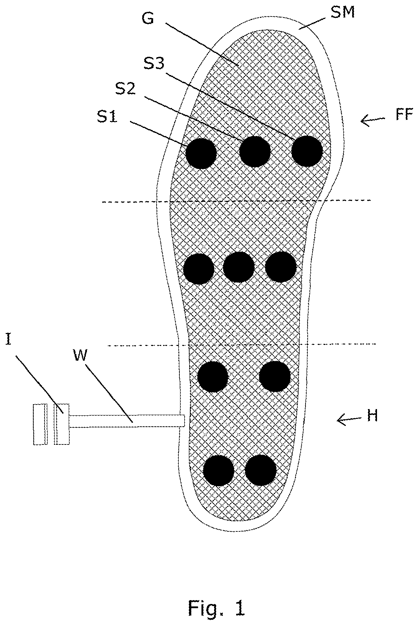

Fig. 1 illustrates the layout of an insole embodiment. A foot shaped platform SM of a sole material such as rubber, polymer, plastic, shock-absorbing synthetic foam etc. which forms the basis of the insole. On top of the platform SM, the black areas indicate stimulation electrodes S1, S2, S3, here shown in a version with a total of 10 stimulation electrodes S1, S2, S3. A ground plane electrode G is indicated as a crossed area, and even though not clear on the sketch, it is understood that the stimulation electrodes S1, S2, S3 are electrically insulated from the ground plane electrode G. To provide good electrical contact with the skin on the foot sole of the person wearing the insole, the ground plane electrode G as well the stimulation electrodes S1, S2, S3 have adhesive surfaces, such as also known from ECG electrodes and the like. Not visible electrical conducting elements ending in a bundle of wires W serve to electrically connect the stimulation electrodes S1, S2, S3 and the ground plane electrode G to respective pins of the single electrical interface plug I, thus allowing easy connection to stimulation control equipment using only one common electrical interface plug I for all electrodes G, S1, S2, S3 that can thus be individually controlled via the plug I. Of course electrode materials, connectors, wires W, and interface plugs I and electrical insulation materials should be able to withstand at least 200 V, preferably at lest 400 V, in order to manage the rather high voltages than can be required for providing stimulation current for reflex-based stimulation.

It appears that the area covered by the ground plane electrode G is significantly larger than the area covered by each of the stimulation electrodes S1, S2, S3, and in the present embodiment the ground plane electrode G covers the majority of the platform area, in fact more than 90% of the platform area, and it is formed as one single area that extends in both forefood, arch of foot, and heel areas. The large area compared to the stimulation electrode area is required to provide the desired painful sensation provoking a withdrawal effect, which is the basis for a reflex-based gait training FET system. Preferably a ratio between area of ground plane electrode and area of one stimulation electrode is more than 10, such as a ratio of 15-20. For cases where the ground plane electrode extends over a large area of the foot, the ratio may be up to such as 50 or even 100. Further, the area of one stimulation electrode is preferably more than 1 cm², such as 1-2 cm², or such as 2-5 cm².

As seen in the layout sketch of Fig. 1, there are groups of a plurality of stimulation electrodes in each of the foot regions: forefoot FF, arch of the foot, and heel H. This spatial distribution of individually addressable stimulation areas will provide a flexible fitting to each person's response, thus the insole is suited for use with a highly automated FET system that is capable of receiving feedback and select the most appropriate stimulation area in response to feedback regarding the movement of the person's leg. Thus, with many distributed stimulation electrodes S1, S2, S3, a good performance can be achieved without the need for individual electrode positioning for each person.

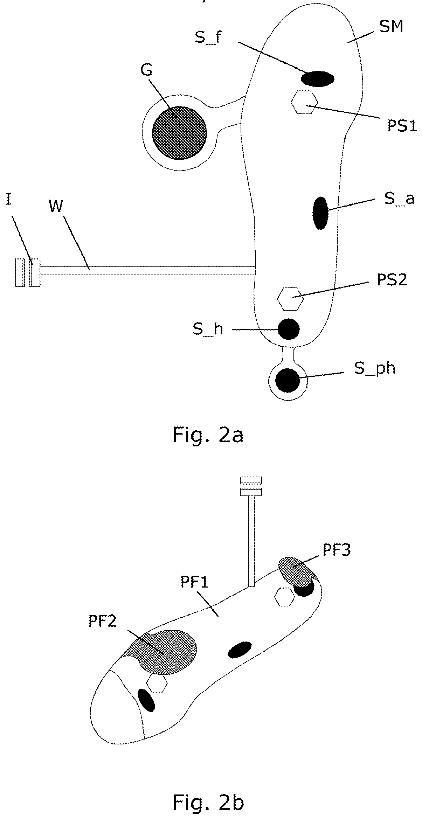

Figs. 2a and 2b illustrate an alternative insole embodiment. Here the platform SM is also foot shaped, but in the illustrated layout only one stimulation electrode is present in each of the foot sole regions, namely a fore foot electrode S_f, an arhc of the foot electrode S_a, and a heel electrode S_h on the main part of the platform SM. In addition, a flap PF3 is attached to the heel part of the main platform part PF1, e.g. integrally manufactured, e.g. monolithically integrated, with the platform SM material, and carries a stimulation electrode S_ph which fits to the posterior part of the person's heel when flapped into place for normal use as is seen in Fig. 2b. In a similar manner a flap PF2 is attached to the main part PF1 of the platform SM. This flap PF2 carries the ground plane electrode G which fits to contact an upper part of the person's foot when flapped into place for normal use. Thus, this embodiment allows stimulation also of the posterior part of the person's heel in addition to the three foot sole regions.

Further, Figs. 2a and 2b illustrate the presence of sensors in the form of two pressure sensitive areas, namely one on the forefoot PS1, and one on the heel PS2. Such pressure sensitive areas can be implemented in various ways as known to the skilled person. The function of the sensors PS1, PS2 is to allow feedback indicative of the position of the person's foot during a step, i.e. primarily to know if part of the person's foot touches the ground or not, and in this way discriminate between gait phases.

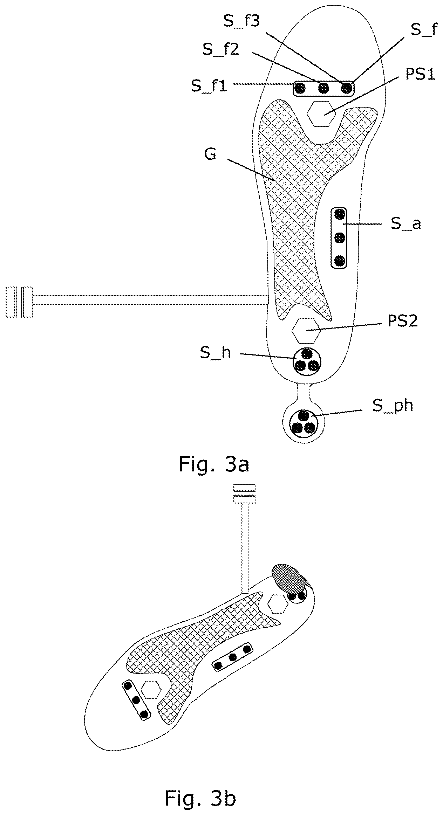

Figs. 3a and 3b illustrate yet another insole embodiment, referring to details in the foregoing embodiments already described. Again a foot shaped platform forms the basis onto which groups of stimulation electrodes, namely one group S_f of three stimulation electrodes S_f1, S_f2, S_f3 in the forefoot region, one group S_a in the arch of foot region, one group in the heel region S_h, and one group S_ph on a heel flap arranged for contact with a posterior part of the person's heel. In addition, two pressure sensitive sensors are positioned in each part of the foot sole, namely one in the forefoot region PS1, and one in the heel region PS2. One large ground plane electrode G extends primarily in the arch of the foot region, and only partly in the forefoot and heel regions. As already mentioned, the groups of a plurality of stimulation electrodes each individually accessible via the electric interface allows for individual adjustment and refining of stimulation, e.g. based on an automated algorithm using feedback from the sensors PS1, PS2.

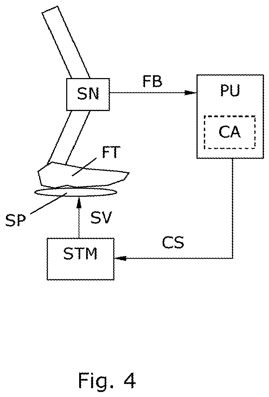

Fig. 4 shows schematically the leg and foot FT of a person together with basic parts of a FET or FES therapy system including a product SP according to the invention. The system includes a stimulator unit STM arranged to apply a stimulation voltage SV to at least a ground plane electrode terminal and a stimulation electrode terminal of the common electrical interface of the insole or shoewear product SP, e.g. in the form of insoles as described in the foregoing. The electrodes of the product SP then accordingly induce a painful sensation on the person's foot FT with the purpose of provoking the person's withdrawal reflex. This reflex results in a withdrawal of the leg, i.e. the leg will initiate a swing phase.

The stimulation voltage SV required, e.g. typically up to voltages within the range 50-400 V though typically around 200 V, in order to generate the required stimulation current to trigger the withdrawal reflex, is applied by the stimulator unit STM which is controlled in response to a control signal CS generated by a processor unit PU which runs a control algorithm CA. This control algorithm CA calculated the control signal CS in response to a feedback signal FB which the processor unit PU receives from a sensor SN which is arranged to sense a parameter, e.g. acceleration of the leg, here illustrated as a goniometer SN sensing the knee angle, and generates the feedback signal FB according to this parameter. The control algorithm CA is preferably designed to adjust the control signal CS so as to obtain a target trajectory for the leg. In the illustrated example, the control algorithm CA may be designed to determine a control signal CS resulting in a stimulation S that would most probably provide a target knee angle of the leg LG as a function of time during one walking step.

The sensor providing the feedback signal FB may in addition to or as an alternative to the shown goniometer SN be a sensor placed in the product SP, such as the illustrated pressure sensors on Figs. 2 and 3, or such sensors may be in the form of simple electric switches mounted between two layers of the product SP. One major purpose of the feedback FB to the control algorithm CA is to provide feedback FB regarding if the foot FT touches the ground or not, so as to provide valuable information to the control algorithm CA with respect to determining when a gait cycle is initiated. It is to be understood that several sensor types may be used to derive a feedback signal which represents a parameter describing the position and/or movement of the leg during the gait. This could be accelerometers, tiltsensors and gyroscopes that are used for estimating the position of the leg and thereby joint angles. E.g. a camera can be used to capture digital images of the leg during the patient walking. Via appropriate image analysis processing on the image, a suitable feedback can be derived, e.g. in the form of one or more of hip, knee, and ankle joint angles and foot distance to ground, or in the form of a more complex parametric description of the leg movement. However, in case only sensor(s) are mounted in the product SP, only one single electric interface is required to connect both stimulation electrodes as well as sensors providing the feedback FB to the processor unit PU.

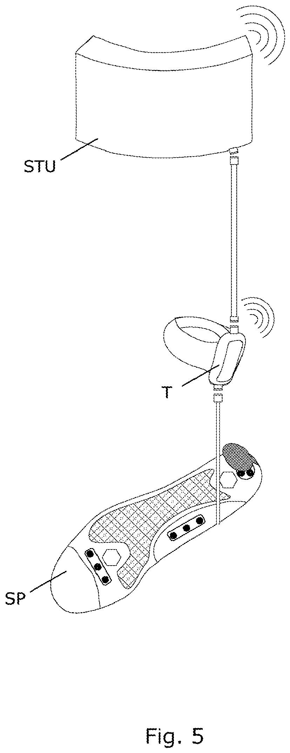

Fig. 5 shows a drawing of equipment for a FET system including an insole product SP, here illustrated as the one from Figs. 3a, 3b. The insole SP is via its electric interface connected to a transmitter T which has a strap for fastening around the person's lower leg. The transmitter is connected to transmit a wireless RF signal indicative of the pressure sensed by the pressure sensors in the insole, (and potentially also from sensors mounted together with the lower leg transmitter e.g. accelometers, tiltsensors, and gyroscopes) and thus such signal can be used as input to a stimulation unit STU or a control unit, e.g. a laptop computer, in a reflex-based gait therapy system. A stimulation unit STU is also connected via a wire to the common electrical interface I of the insole SP, and this unit generates the electrical stimulation signal to the appropriate one(s) of stimulation electrodes on the insole SP in response to an incoming wireless RF signal from a control system, e.g. a laptop computer. The stimulation unit STU may be fastened to the person's waist belt or it may be fastened to the person with a dedicated strap.

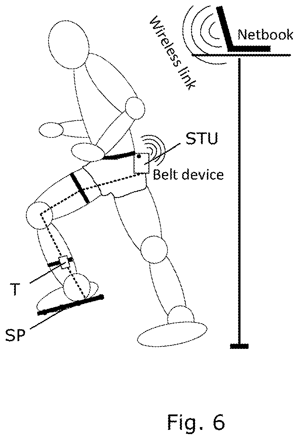

Fig. 6 illustrates the same equipment SP, T, STU as shown in Fig. 5, but here the equipment is sketched mounted on a person, and the wireless link to a netbook computer is also indicated. Thus, the control system of the FET system with feedback is implemented in software on the netbook computer, i.e. the control algorithm is run on the computer which wirelessly controls stimulation via the stimulation unit STU by transmitting a control signal which the unit STU translates into electric signal(s) which is applied to the appropriate one(s) of the stimulation electrodes.

It is to be understood that in all the illustrated embodiments, goniometers may be entirely replaced by sensor(s) in the form of accelerometer(s) and/or gyroscope(s). Especially, it may be preferrred to build in such accelerometer(s) into a part of an insole.

To sum up, the invention provides a product, e.g. an insole for a shoe, fitting onto a person's foot sole based on a platform (SM) of a flexible and electrically isolating material. Multiple stimulation electrodes (S1, S2, S3) with adhesive stimulation areas of at least 1 cm² are spatially distributed on the platform (SM). A conducting and adhesive ground plane electrode (G) with at least an area of 10 times a stimulation electrode area is arranged for contacting the person's foot. A common electrical interface (I) comprising a plug or socket with externally accessible electrical connectors to allow external individual electrical access to the plurality of stimulation electrode areas (S1, S2, S3) and to the ground plane electrode (G). The common electrical interface is capable of handling at least a voltage of 200 V, preferably 400 V. Such product is suited for reflex-based gait therapy, where the person's withdrawal reflex is triggered by a painful stimulation of the person's foot sole. Many distributed stimulation electrodes allow individual fitting for optimal stimulation, and low cost sensors built into the product may be used to provide feedback regarding the gait phase, i.e. the product is a one-time use insole suited for easy and hygienic gait therapy in a clinic.

The invention is defined in the appended claims. Aspects, embodiments and examples disclosed herein which do not fall within the scope of the appended claims do not form part of the invention, and are merely provided for illustrative purposes.

## Claims

1. A product shaped and configured to fit onto a person's foot sole, the product comprising
- a platform (SM) of a flexible and electrically isolating material,
- a plurality of separate stimulation electrodes (S1, S2, S3) each having an adhesive stimulation area of at least 1 cm², wherein the stimulation electrodes (S1, S2, S3) are spatially distributed on the platform (SM) so as to be able to stimulate different zones of the person's foot sole,
- at least one electrically conducting and adhesive ground plane electrode (G) configured to contact the person's foot, wherein a total area of the ground plane electrode (G) is at least 10 times greater than the area of one stimulation electrode (S1, S2, S3),
- a common electrical interface (I), electrically connected to the plurality of separate stimulation electrodes (S1, S2, S3) and the ground plane electrode (G), the common electrical interface (I) comprising a plug or socket with externally accessible electrical connectors configured to allow external individual electrical access to the plurality of stimulation electrode areas (S1, S2, S3) and to the ground plane electrode (G), and
- a set of electrical conductors (W) configured to interconnect the plurality of stimulation electrodes (S1, S2, S3) and the ground plane electrode (G) with the electrical connectors of the common electrical interface (I), wherein the set of electrical conductors (W) are configured to withstand a voltage of more than 200 V between the ground plane electrode (G) and the plurality of stimulation electrodes (S1, S2, S3).

2. Product according to claim 1, wherein the product is one of: a replaceable insole arranged to fit into a shoe, a slipper, and a shoe.

3. Product according to any of the preceding claims, wherein the product is a replaceable insole arranged to fit into a person's shoe, and wherein the platform comprises an upper layer and a lower layer with the set of electrical conductors arranged between the upper and lower layers, and wherein the stimulation electrodes extend through holes in the upper layer.

4. Product according to claim 3, wherein at least the upper layer is made of a reuseable material.

5. Product according to any of the preceding claims, wherein the plurality of stimulation electrodes are spatially distributed on the platform (SM) so as to enable stimulation of a plurality of different positions of each one of the person's foot sole areas: heel, fore foot, and mid foot.

6. Product according to any of the preceding claims, wherein the ground plane (G) extends spatially so as to enable contact to a plurality of the person's foot regions: heel of foot sole, fore foot of foot sole, mid foot of foot sole, back of heel, and upper part of foot.

7. Product according to any of the preceding claims, comprising an additional platform element structurally linked to the platform (SM), wherein the additional platform element comprises a stimulation electrode or a ground plane electrode in electrical connection with said common electrical interface (I).

8. Product according to claim 7, wherein the additional platform element is connected to the platform (SM) so as to enable the stimulation electrode or the ground plane electrode to be in contact with an upper part of or a back of the heel of the person's foot.

9. Product according to any of the preceding claims, comprising a sensor arranged to provide a feedback indicative of a person's gait phase upon the person walking.

10. Product according to claim 9, wherein the sensor is electrically connected to one or more electrical connectors within said common electrical interface (I), and wherein at least one sensor is spatially arranged in relation to the platform (SM) and configured to enable sensing of the person's heel touching the ground.

11. Product according to any of claims 9 or 10, wherein the sensor comprises a plurality of spatially distributed sensor elements, in different regions of the platform so as to enable sensing of respective areas of the person's foot touching the ground.

12. Product according to any of the preceding claims, wherein the set of electrical conductors (W) comprises a flexible PCB spatially extending within the platform (SM) so as to electrically connect each of the plurality of stimulation electrodes (S1, S2, S3) and the respective electrical connectors of the common electrical interface (I).

13. Product according to any of the preceding claims, wherein the platform (SM) forms part of a footwear, such as a shoe, or a slipper.

14. System arranged for gait training, such as for rehabilitation of a person after a stroke, the system comprising
- a product according to any of claims 1-13,
- a stimulation unit arranged for connection to the common electrical interface (I) of the product, wherein the stimulation unit is arranged to generate an electrical stimulation voltage between the ground plane electrode and at least one of the stimulation electrodes, wherein the electrical stimulation voltage is high enough to induce a painful sensation on a foot (FT) of a person wearing the product with the purpose of provoking the person's withdrawal reflex.

15. System according to claim 14, comprising
- a sensor arranged to sense a parameter representative of a movement of the person's leg and to generate a feedback signal accordingly, and
- a processor unit operationally connected to the stimulation unit and to the sensor, the processor unit comprising a processor running a control algorithm configured to generate a control signal to the stimulation unit in response to the feedback signal.

## Patentansprüche

1. Produkt, das so geformt und konfiguriert ist, dass es auf die Fußsohle einer Person passt, das Produkt umfassend
- eine Plattform (SM) aus einem flexiblen und elektrisch isolierenden Material,
- mehrere separate Stimulationselektroden (S1, S2, S3), die jeweils eine haftende Stimulationsfläche von mindestens 1 cm² aufweisen, wobei die Stimulationselektroden (S1, S2, S3) räumlich auf der Plattform (SM) verteilt sind, um verschiedene Zonen der Fußsohle der Person stimulieren zu können,
- mindestens eine elektrisch leitende und haftende Masseelektrode (G), die so konfiguriert ist, dass sie den Fuß der Person berührt, wobei die Gesamtfläche der Masseelektrode (G) mindestens zehnmal größer ist als die Fläche einer Stimulationselektrode (S1, S2, S3),
- eine gemeinsame elektrische Schnittstelle (I), die elektrisch mit mehreren separaten Stimulationselektroden (S1, S2, S3) und der Masseelektrode (G) verbunden ist, wobei die gemeinsame elektrische Schnittstelle (I) einen Stecker oder eine Buchse mit von außen zugänglichen elektrischen Anschlüssen umfasst, die so konfiguriert sind, dass sie einen externen individuellen elektrischen Zugang zu den mehreren Stimulationselektrodenbereichen (S1, S2, S3) und zu der Masseelektrode (G) ermöglichen, und
- einen Satz elektrischer Leiter (W), die so konfiguriert sind, dass sie mehrere Stimulationselektroden (S1, S2, S3) und die Masseelektrode (G) mit den elektrischen Verbindern der gemeinsamen elektrischen Schnittstelle (I) verbinden, wobei der Satz elektrischer Leiter (W) so konfiguriert ist, dass er einer Spannung von mehr als 200 V zwischen der Masseelektrode (G) und den mehreren Stimulationselektroden (S1, S2, S3) standhält.

2. Produkt nach Anspruch 1, wobei es sich bei dem Produkt um eines der folgenden Produkte handelt: eine austauschbare Einlegesohle, die in einen Schuh passt, einen Hausschuh und einen Schuh.

3. Produkt nach einem der vorhergehenden Ansprüche, wobei das Produkt eine austauschbare Einlegesohle ist, die so angeordnet ist, dass sie in den Schuh einer Person passt, und wobei die Plattform eine obere Schicht und eine untere Schicht umfasst, wobei der Satz elektrischer Leiter zwischen der oberen und der unteren Schicht angeordnet ist, und wobei die Stimulationselektroden durch Löcher in der oberen Schicht verlaufen.

4. Produkt nach Anspruch 3, wobei mindestens die obere Schicht aus einem wiederverwendbaren Material besteht.

5. Produkt nach einem der vorhergehenden Ansprüche, wobei die mehreren Stimulationselektroden räumlich auf der Plattform (SM) verteilt sind, um die Stimulation mehrerer verschiedener Positionen jedes Fußsohlenbereichs der Person zu ermöglichen: Ferse, Vorderfuß und Mittelfuß.

6. Produkt nach einem der vorhergehenden Ansprüche, wobei sich die Grundplatte (G) räumlich so erstreckt, dass sie den Kontakt zu mehreren Fußbereichen der Person ermöglicht: Ferse der Fußsohle, Vorderfuß der Fußsohle, Mittelfuß der Fußsohle, Fersenrücken und oberer Teil des Fußes.

7. Produkt nach einem der vorhergehenden Ansprüche, umfassend ein zusätzliches Plattformelement, das strukturell mit der Plattform (SM) verbunden ist, wobei das zusätzliche Plattformelement eine Stimulationselektrode oder eine Masseelektrode in elektrischer Verbindung mit der gemeinsamen elektrischen Schnittstelle (I) umfasst.

8. Produkt nach Anspruch 7, wobei das zusätzliche Plattformelement so mit der Plattform (SM) verbunden ist, dass die Stimulationselektrode oder die Masseelektrode mit dem oberen Teil oder der Rückseite der Ferse des Fußes der Person in Kontakt kommen kann.

9. Produkt nach einem der vorhergehenden Ansprüche, umfassend einen Sensor, der so angeordnet ist, dass er ein Feedback über die Gangphase einer Person beim Gehen bereitstellt.

10. Produkt nach Anspruch 9, wobei der Sensor elektrisch mit einem oder mehreren elektrischen Verbindern innerhalb der gemeinsamen elektrischen Schnittstelle (I) verbunden ist, und wobei mindestens ein Sensor räumlich in Bezug auf die Plattform (SM) angeordnet und so konfiguriert ist, dass er das Erfassen der den Boden berührenden Ferse der Person ermöglicht.

11. Produkt nach einem der Ansprüche 9 oder 10, wobei der Sensor mehrere räumlich verteilte Sensorelemente in verschiedenen Bereichen der Plattform umfasst, um das Erfassen der jeweiligen Bereiche des Fußes der Person, die den Boden berühren, zu ermöglichen.

12. Produkt nach einem der vorhergehenden Ansprüche, wobei der Satz elektrischer Leiter (W) eine flexible Leiterplatte umfasst, die sich räumlich innerhalb der Plattform (SM) erstreckt, um jede der mehreren Stimulationselektroden (S1, S2, S3) und die jeweiligen elektrischen Anschlüsse der gemeinsamen elektrischen Schnittstelle (I) elektrisch zu verbinden.

13. Produkt nach einem der vorhergehenden Ansprüche, wobei die Plattform (SM) Teil eines Schuhwerks, wie etwa eines Schuhs oder eines Hausschuhs, ist.

14. System, das zur Gangschulung angeordnet ist, wie etwa. zur Rehabilitation einer Person nach einem Schlaganfall, das System umfassend
- ein Produkt nach einem der Ansprüche 1-13,
- eine Stimulationseinheit, die zur Verbindung mit der gemeinsamen elektrischen Schnittstelle (I) des Produkts angeordnet ist, wobei die Stimulationseinheit angeordnet ist, um eine elektrische Stimulationsspannung zwischen der Masseelektrode und mindestens einer der Stimulationselektroden zu erzeugen, wobei die elektrische Stimulationsspannung hoch genug ist, um eine schmerzhafte Empfindung an einem Fuß (FT) einer Person, die das Produkt trägt, zu erzeugen, mit dem Zweck, den Rückzugsreflex der Person hervorzurufen.

15. System nach Anspruch 14, umfassend
- einen Sensor, der so angeordnet ist, dass er einen Parameter erfasst, der für eine Bewegung des Beins der Person repräsentativ ist, und dementsprechend ein Feedbacksignal erzeugt, und
- eine Prozessoreinheit, die betriebsmäßig mit der Stimulationseinheit und dem Sensor verbunden ist, wobei die Prozessoreinheit einen Prozessor umfasst, der einen Steueralgorithmus ausführt, der so konfiguriert ist, dass er als Reaktion auf das Feedbacksignal ein Steuersignal für die Stimulationseinheit erzeugt.

## Revendications

1. Produit formé et configuré pour s'adapter à une plante de pied d'une personne, le produit comprenant :
- une plateforme (SM) en un matériau isolant électriquement et flexible,
- une pluralité d'électrodes de stimulation séparées (S1, S2, S3) ayant chacune une zone de stimulation adhésive d'au moins 1 cm², dans lequel les électrodes de stimulation (S1, S2, S3) sont réparties spatialement sur la plateforme (SM) de manière à pouvoir stimuler différentes zones de la plante du pied de la personne,
- au moins une électrode de plan de masse adhésive et électroconductrice (G) configurée pour contacter le pied de la personne, dans lequel une superficie totale de l'électrode de plan de masse (G) est au moins 10 fois supérieure à la superficie d'une électrode de stimulation (S1, S2, S3),
- une interface électrique commune (I) raccordée électriquement à la pluralité des électrodes de stimulation séparées (S1, S2, S3) et à l'électrode de plan de masse (G), l'interface électrique commune (I) comprenant une fiche ou une prise avec des connecteurs électriques accessibles de manière externe configurés pour permettre un accès électrique individuel externe à la pluralité des superficies d'électrodes de stimulation (S1, S2, S3) et à l'électrode de plan de masse (G), et
- un ensemble de conducteurs électriques (W) configurés pour interconnecter la pluralité des électrodes de stimulation (S1, S2, S3) et l'électrode de plan de masse (G) avec les connecteurs électriques de l'interface électrique commune (I), dans lequel l'ensemble de conducteurs électriques (W) sont configurés pour résister à une tension de plus de 200 V entre l'électrode de plan de masse (G) et la pluralité des électrodes de stimulation (S1, S2, S3).

2. Produit selon la revendication 1, dans lequel le produit est l'un(e) parmi : une semelle intérieure remplaçable étudiée pour s'adapter dans une chaussure, un chausson, et une chaussure.

3. Produit selon l'une quelconque des revendications précédentes, dans lequel le produit est une semelle intérieure remplaçable étudiée pour s'adapter dans une chaussure d'une personne, et dans lequel la plateforme comprend une couche supérieure et une couche inférieure avec l'ensemble de conducteurs électriques disposés entre les couches supérieure et inférieure, et dans lequel les électrodes de stimulation s'étendent à travers des trous dans la couche supérieure.

4. Produit selon la revendication 3, dans lequel au moins la couche supérieure est constituée d'un matériau réutilisable.

5. Produit selon l'une quelconque des revendications précédentes, dans lequel la pluralité des électrodes de stimulation sont réparties spatialement sur la plateforme (SM) de manière à permettre une stimulation d'une pluralité de différents positions de chacune des superficies de la plante du pied de la personne : talon, avant-pied, et médio-pied.

6. Produit selon l'une quelconque des revendications précédentes, dans lequel le plan de masse (G) s'étend spatialement de manière à permettre un contact avec une pluralité de zones du pied d'une personne : talon de la plante du pied, avant-pied de la plante du pied, médio-pied de la plante du pied, arrière du talon, et partie supérieure du pied.

7. Produit selon l'une quelconque des revendications précédentes, comprenant un élément de plateforme supplémentaire relié structurellement à la plateforme (SM), dans lequel l'élément de plateforme supplémentaire comprend une électrode de stimulation ou une électrode de plan de masse en connexion électrique avec ladite interface électrique commune (I).

8. Produit selon la revendication 7, dans lequel l'élément de plateforme supplémentaire est connecté à la plateforme (SM) de manière à permettre à l'électrode de stimulation ou à l'électrode de plan de masse d'être en contact avec une partie supérieure du talon, ou un arrière de celui-ci, du pied de la personne.

9. Produit selon l'une quelconque des revendications précédentes, comprenant un capteur étudié pour fournir un feedback indicatif d'une phase de déambulation de la personne lorsque la personne marche.

10. Produit selon la revendication 9, dans lequel le capteur est connecté électriquement à un ou plusieurs connecteurs électriques dans ladite interface électrique commune (I), et dans lequel au moins un capteur est disposé spatialement en rapport avec la plateforme (SM) et est configuré pour permettre de détecter le talon de la personne touchant le sol.

11. Produit selon l'une quelconque des revendications 9 ou 10, dans lequel le capteur comprend une pluralité d'éléments de détection répartis spatialement dans différentes zones de la plateforme de manière à permettre une détection de superficies respectives du pied de la personne touchant le sol.

12. Produit selon l'une quelconque des revendications précédentes, dans lequel l'ensemble de conducteurs électriques (W) comprend une carte de circuit imprimé flexible s'étendant spatialement à l'intérieur de la plateforme (SM) de manière à connecter électriquement chacune de la pluralité des électrodes de stimulation (S1, S2, S3) et les connecteurs électriques respectifs de l'interface électrique commune (I).

13. Produit selon l'une quelconque des revendications précédentes, dans lequel la plateforme (SM) forme une partie d'un article chaussant, tel une chaussure ou un chausson.

14. Système étudié pour l'entraînement à la déambulation, tel que pour la réhabilitation d'une personne après un accident vasculaire cérébral, le système comprenant :
- un produit selon l'une quelconque des revendications 1-13,
- une unité de stimulation étudiée pour la connexion à l'interface électrique commune (I) du produit, dans lequel l'unité de stimulation est étudiée pour générer une tension de stimulation électrique entre l'électrode de plan de masse et au moins l'une des électrodes de stimulation, dans lequel la tension de stimulation électrique est suffisamment élevée pour induire une sensation douloureuse d'un pied (FT) d'une personne portant le produit avec pour objectif de provoquer un réflexe de retrait de la personne.

15. Système selon la revendication 14, comprenant :
- un capteur étudié pour détecter un paramètre représentatif d'un mouvement de la jambe de la personne et pour générer un signal de feedback en conséquence, et
- une unité de traitement connectée à l'unité de stimulation et au capteur, l'unité de traitement comprenant un processeur exécutant un algorithme de commande configuré pour générer un signal de commande pour l'unité de stimulation en réponse au signal de feedback.
